(19) Europäisches Patentamt
European Patent Office
Office européen des brevets

(11) Veröffentlichungsnummer: **0 173 316**
**A2**

# (12) EUROPÄISCHE PATENTANMELDUNG

(21) Anmeldenummer: **85110827.4**

(22) Anmeldetag: **19.08.85**

(51) Int. Cl.⁴: **C 07 D 239/42**, C 07 D 239/46, C 07 D 239/52, C 07 D 213/75, A 01 N 47/36 // C07D417/12, C07D251/52, C07D251/46, C07D239/48

(30) Priorität: **30.08.84 DE 3431930**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Hoehe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Lanhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen (DE)**
Erfinder: **Riebel, Hans-Joachem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Koeln 80 (DE)**
Erfinder: **Schmidt, Robert R., Dr., Im Waldwinkel 110, D-5060 Bergisch-Gladbach 2 (DE)**

## (54) 1-(2-Oxyaminosulfonylphenylsulfonyl)-3-heteroaryl-iso-(thio)-harnstoffe.

(57) Die Erfindung betrifft neue 1-(2-Oxyaminosulfonylphenylsulfonyl) -3- heteroaryl-iso(thio)-harnstoffe der allgemeinen Formel (I)

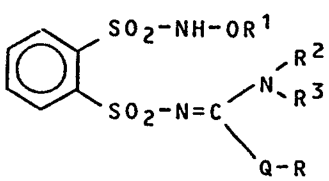

(I)

in welcher

R für einen gegebenenfalls substituierten Rest aus der Reihe Alkyl, Alkenyl, Alkinyl, Cycloalkyl-alkyl, Cycloalkyl, Aralkyl, Aryl und Hetaryl steht,

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht,

$R^3$ für einen gegebenenfalls substituierten und/oder gegebenenfalls anellierten sechsgliedrigen aromatischen Heterocyclus welcher wenigstens ein Stickstoffatom enthält, steht und

Q für Sauerstoff oder Schwefel steht, sowie Addukte von Verbindungen der Formel (I) mit starken Säuren,

Verfahren zu ihrer Herstellung und ihre Verwendung als Herbizide.

EP 0 173 316 A2

ACTORUM AG

0 173 316

BAYER AKTIENGESELLSCHAFT
Konzernverwaltung RP
Patentabteilung

5090 Leverkusen, Bayerwerk
Bi/sam.
IVb

## 1-(2-Oxyaminosulfonylphenylsulfonyl)-3-heteroaryl-iso-(thio)-harnstoffe

Die Erfindung betrifft neue 1-(2-Oxyaminosulfonylphenylsulfonyl)-3-heteroaryl-iso-(thio)-harnstoffe, erfinderische Verfahren zu deren Herstellung und deren Verwendung als Herbizide.

Verschiedene Isoharnstoffe und Isothioharnstoffe sind als potentielle Herbizide bekannt geworden, haben jedoch bisher als Mittel zur Unkrautbekämpfung und/oder Regulierung des Pflanzenwachstums keine größere Bedeutung erlangt (vergl. DE-AS 1 138 039 und GB-PS 1 202 736).

Es wurden nun neue 1-(2-Oxyaminosulfonylphenylsulfonyl)-3-heteroaryl-iso-(thio)-harnstoffe der allgemeinen Formel (I)

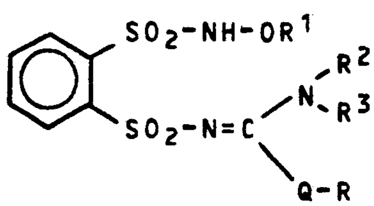

(I)

in welcher

R für einen gegebenenfalls substituierten Rest aus der Reihe Alkyl, Alkenyl, Alkinyl, Cycloalkyl-alkyl, Cycloalkyl, Aralkyl, Aryl und Hetaryl steht,

$R^1$ für einen gegebenenfalls substituierten Rest aus der Reihe Alkyl, Alkenyl, Alkinyl, Cycloalkyl, Cycloalkyl-alkyl, Aralkyl und Aryl steht,

$R^2$ für Wasserstoff oder für einen gegebenenfalls substituierten Rest aus der Reihe Alkyl, Alkenyl, Alkinyl und Aralkyl steht,

$R^3$ für einen gegebenenfalls substituierten und/oder gegebenenfalls anellierten sechsgliedrigen aromatischen Heterocyclus,welcher wenigstens ein Stickstoffatom enthält, steht und

Q für Sauerstoff oder Schwefel steht,

sowie Addukte der Verbindungen der Formel (I) mit starken Säuren gefunden.

Man erhält die neuen Verbindungen der Formel (I), wenn man

(a) Benzodisultame der Formel (II)

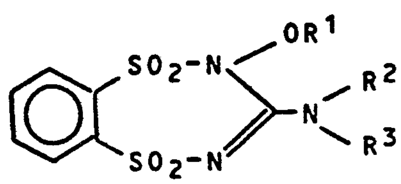

(II)

in welcher

$R^1$,$R^2$ und $R^3$ die oben angegebenen Bedeutungen haben,

mit Verbindungen der Formel (III)

$$M - Q - R \qquad (III)$$

in welcher

Q und R die oben angegebenen Bedeutungen haben und

M für Wasserstoff oder ein Metalläquivalent steht,

gegebenenfalls in Gegenwart von Basen und gegebenenfalls in Gegenwart von Verdünnungsmitteln umsetzt und gegebenenfalls die hierbei erhaltenen Produkte mit Säuren behandelt,

oder, wenn man

(b) Oxyguanidin-Derivate der Formel (IV)

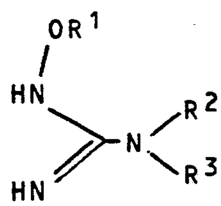

(IV)

in welcher $R^1$, $R^2$ und $R^3$ die oben angegebenen Bedeutungen haben,

zunächst mit Benzol-1,2-disulfonsäuredichlorid der Formel (V)

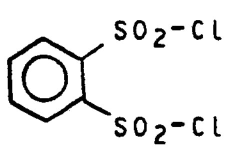

(V)

in Gegenwart von Säureakzeptoren und gegebenenfalls in Gegenwart von Verdünnungsmitteln umsetzt und anschließend, ohne Isolierung des Reaktionsproduktes der Formel (II)

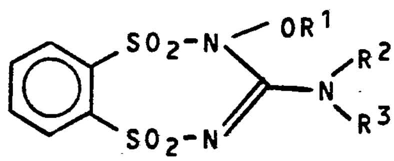

(II)

in welcher

$R^1$, $R^2$ und $R^3$ die oben angegebenen Bedeutungen haben,

die Reaktionsmischung mit Verbindungen der Formel (III)

$$M - Q - R \qquad (III)$$

in welcher

Q und R die oben angegebenen Bedeutungen haben und

M für Wasserstoff oder ein Metalläquivalent steht,

gegebenenfalls in Gegenwart von Basen und in Gegenwart von Verdünnungsmitteln umsetzt und gegebenenfalls die hierbei erhaltenen Produkte mit Säuren behandelt.

Die neuen Sulfonyliso(thio)harnstoff-Derivate der Formel (I) und ihre Addukte mit starken Säuren zeichnen sich durch starke herbizide Wirksamkeit aus.

Ueberraschenderweise zeigen die neuen Verbindungen der Formel (I) erheblich bessere herbizide Wirkung als vorbekannte Isoharnstoff- bzw. Isothioharnstoff-Derivate gleicher Wirkungsrichtung.

Es ist weiter als überraschend anzusehen, daß die erfindungsgemäßen Verbindungen der Formel (I) durch selektive Ringöffnung von Heterocyclen der Formel (II) hergestellt werden können, da neben dieser neuartigen Reaktion auch andere Ringöffnungen, beispielsweise durch Angriff an den Sulfonyl-Gruppierungen zu erwarten waren.

Gegenstand der Erfindung sind vorzugsweise Verbindungen der Formel (I), in welcher

R für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] steht oder

für einen Phenylrest steht, welcher gegebenenfalls substituiert ist durch einen oder mehrere Reste aus der Reihe
Halogen [wie insbesondere Fluor, Chlor, Brom und Jod],

Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1$-$C_4$-Alkoxy-carbonyl-amino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyl-oxy, $C_1$-$C_4$-Alkoxy-carbonyl-oxy, $C_1$-$C_4$-Alkyl-amino-carbonyl-oxy und Di-($C_1$-$C_4$-alkyl)-amino-carbonyl-oxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; worin weiter

R für einen fünf- oder sechsgliedrigen heteroaromatischen Ring steht, welcher 1 bis 3 Stickstoffatome und/oder ein Sauerstoff- oder Schwefelatom enthält und welcher gegebenenfalls benzanelliert ist und/oder durch Fluor, Chlor, Brom, Cyano, Nitro, $C_1$-$C_3$-Alkyl oder $C_1$-$C_3$-Alkoxy [wobei letztere gegebenenfalls durch Fluor und/oder Chlor substituiert sind] substituiert ist; worin weiter

$R^1$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht, in welcher weiter

$R^2$ für Wasserstoff, $C_1$-$C_4$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl,

$C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl oder Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] steht, in welcher ferner

$R^3$ für den Rest 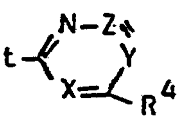 steht, worin

$R^4$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht,

X für Stickstoff oder eine Methin-brücke (CH) steht,

Y für Stickstoff oder eine gegebenenfalls substituierte Methin-brücke C-$R^5$ steht, wobei

$R^5$ für Wasserstoff, Fluor, Chlor, Brom oder $C_1$-$C_4$-Alkyl steht, und

Z für Stickstoff oder eine gegebenenfalls substituierte Methin-brücke C-$R^6$ steht, wobei

$R^6$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht und

Q für Sauerstoff oder Schwefel steht.

Gegenstand der Erfindung sind weiter vorzugsweise Addukte von Verbindungen der Formel (I) - wie vorausgehend definiert - mit Halogenwasserstoffsäuren, wie Hydrogenfluorid, Hydrogenchlorid, Hydrogenbromid, Hydrogeniodid, mit Schwefelsäure, mit gegebenenfalls durch Fluor und/oder Chlor substituierten Alkansulfonsäuren mit 1 bis 4 Kohlenstoffatomen oder auch Benzol- oder Naphtalinsulfonsäuren, welche gegebenenfalls durch Fluor, Chlor, Brom oder Methyl substituiert sind.

Gegenstand der Erfindung sind insbesondere Verbindungen der Formel (I), in welcher

R für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenylethyl oder Benzyl [welches gegebenenfalls durch Fluor, Chlor, Nitro, Cyano, Methyl, Methoxy oder Methoxy-carbonyl substituiert ist] steht, oder

für einen Phenylrest steht, welcher gegebenenfalls substituiert ist durch einen oder zwei Reste aus der Reihe Fluor, Chlor, Brom, Jod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pyridoxy [welches gegebenenfalls durch Chlor und/oder Trifluormethyl substituiert ist], oder welcher gegebenenfalls benzanelliert ist; worin weiter

$R^1$ für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenylethyl oder Benzyl [welches gegebenenfalls durch Fluor, Chlor, Nitro, Cyano, Methyl, Methoxy oder Methoxy-carbonyl substituiert ist] steht,

$R^2$ für Wasserstoff steht und

$R^3$ für den Rest 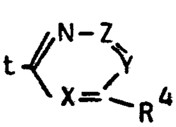 steht, worin

$R^4$ für Chlor, Methyl, Methoxy oder Ethoxy steht,

X für Stickstoff steht,

Y für eine Methin-brücke (CH) steht, und

Z für eine gegebenenfalls substituierte Methin-brücke $C-R^6$ steht, wobei

$R^6$ für Wasserstoff, Chlor, Methyl, Methoxy, Ethoxy, Methylthio, Ethylthio, Dimethylamino oder Diethylamino steht und

Q für Sauerstoff oder Schwefel steht.

Die beim erfindungsgemäßen Verfahren (a) ablaufende chemische Reaktion kann beispielsweise durch folgendes Formelschema skizziert werden:

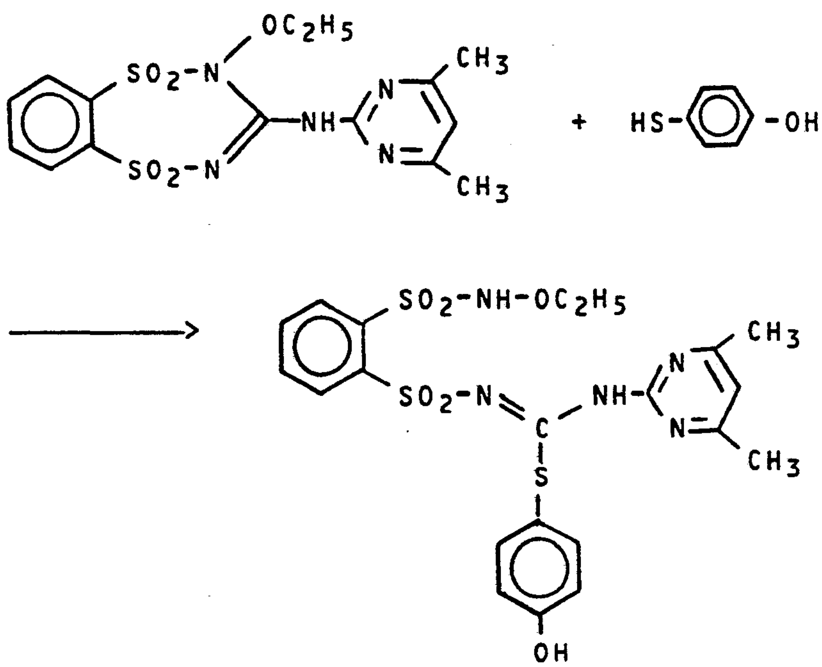

Die beim erfindungsgemäßen Verfahren (b) ablaufenden chemischen Reaktion können beispielsweise durch folgendes Formelschema skizziert werden:

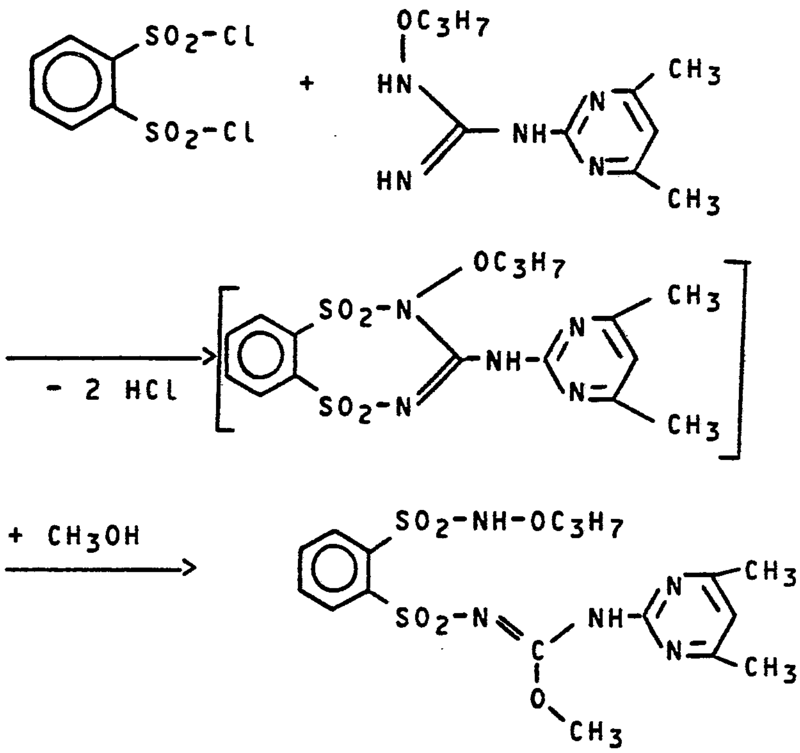


Die beim erfindungsgemäßen Verfahren (a) als Ausgangsstoffe zu verwendenden Benzodisultame sind durch die Formel (II) allgemein definiert.

In Formel (II) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für Ausgangsstoffe der Formel (II) sind in der nachstehenden Tabelle 1 aufgeführt.

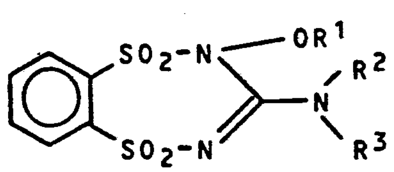

(II)

Tabelle 1: Beispiele für Ausgangsstoffe der Formel (II)

| $R^1$ | $R^2$ | $R^3$ |
|---|---|---|
| $-C_2H_5$ | H | 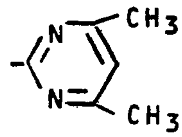 |
| $-C_3H_7$-iso | H | 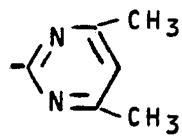 |
| $-C_3H_7$-n | H | 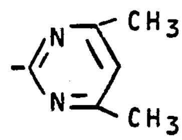 |
| $-C_4H_9$-n | H | 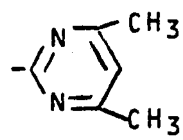 |
| $-C_8H_{17}$-n | H | 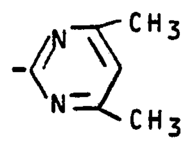 |
| $-CH_2-$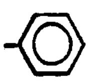 | H | 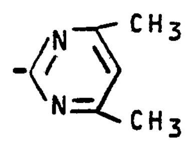 |
| $-CH_2CH_2-$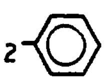 | H | 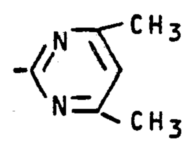 |

Die Verbindungen der Formel (II) sind bisher nicht in der Literatur beschrieben. Man erhält die Verbindungen der Formel (II), wenn man Benzol-1,2-disulfonsäure-dichlorid der Formel (V)

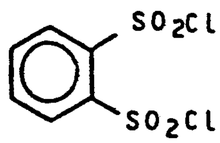

(V)

mit Oxyguanidin-Derivaten der Formel (IV)

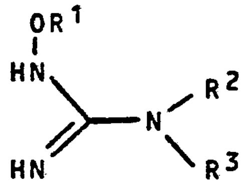

(IV)

in welcher

$R^1$, $R^2$ und $R^3$ die oben angegebene Bedeutung haben,

in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid, Chloroform, Tetrahydrofuran oder Dioxan, bei Temperaturen zwischen -30°C und +50°C umsetzt.

Die Aufarbeitung kann nach üblichen Methoden erfolgen, beispielsweise durch Einengen, Aufnehmen des Rückstandes in Methylenchlorid, Waschen mit verdünnter Salzsäure und mit Wasser, Abtrennen, Trocknen, Filtrieren und Einengen der organischen Phase, wobei die Produkte der Formel (II) im Rückstand verbleiben.

Das als Ausgangsstoff zu verwendende Benzol-1,2-disulfonsäure-dichlorid der Formel (V ) ist bereits bekannt (vgl. J.Org.Chem. 31, (1966), 3289-3292).

Die weiter als Ausgangsstoffe zu verwendenden Oxyguanidin-Derivate sind durch die Formel (IV) allgemein definiert. In Formel (IV) haben $R^1$, $R^2$ und $R^3$ vorzugsweise bzw. insbesondere die gleichen Bedeutungen, wie sie oben im Rahmen der Substituenten-definition der Formel (I) vorzugsweise bzw. als insbesondere bevorzugt angegeben sind.

Als Ausgangsstoffe der Formel (IV) seien beispielsweise genannt:

N'-(4-Methyl-pyrimidin-2-yl)-, N'-(4,6-Dimethyl-pyrimidin-2-yl)-,N'-(4-Methoxy-6-methyl-pyrimidin-2-yl)-,N'-(4-Ethoxy-6-methyl-pyrimidin-2-yl)-, N'-(4-Chlor-6-methoxy-pyrimidin-2-yl)-,N'-(4-Chlor-6-ethoxy-pyrimidin-2-yl)-,N'-(4-Chlor-6-dimethylamino-pyrimidin-2-yl)-,N'-(4-Methyl-6-methylthio-pyrimidin-2-yl)- und N'-(4-Dimethylamino-6-methyl-pyrimidin-2-yl)-N''-methoxy-guanidin, -N''-ethoxy-guanidin,-N''-propoxy-guanidin, -N''-isopropoxyguanidin, -N''-butoxy-guanidin, -N''-isobutoxy-guanidin, -N''-sec.-butoxy-guanidin, -N''-pentoxy-guanidin, -N''-isopentoxy-guanidin, -N''-hexyloxy-guanidin, -N''-octyloxy-guanidin, -N''-allyloxy-guanidin, -N''-(2-chlor-ethoxy)-guanidin, -N''-(2-fluor-ethoxy)-guanidin, -N''-(2-chlor-propoxy)-guanidin, -N''-(2-fluor-propoxy)-guanidin, -N''-(3-chlor-propoxy)-guanidin, -N''-(4-chlor-butoxy)-guanidin, -N''-methoxycarbonylmethoxy-guanidin, -N''-ethoxycarbonyl-methoxy-guanidin, -N''-(1-methoxycarbonyl-ethoxy)-guanidin, -N''-(1-ethoxycarbonylethoxy)-guanidin, -N''-dimethylaminocarbonylmethoxy-guanidin, -N''-(2-phenyl-ethoxy)-guanidin, -N''-phenoxy-guanidin, -N''-(4-methyl-benzyloxy)-guanidin, -N''-(4-fluor-benzyloxy)-guanidin, -N''-(4-chlor-benzyloxy)-guanidin, -N''-(4-nitrobenzyloxy)-guanidin, -N''-(2,6-dichlor-benzyloxy)-guanidin, -N''-(4-methoxycarbonyl-benzyloxy)-guanidin und -N''-(4-ethoxycarbonyl-benzyloxy)-guanidin.

Die Ausgangsstoffe der Formel (IV) sind zum Teil bekannt (vgl. J.Chem. Soc. 1962, 3915); zum Teil sind sie Gegenstand einer nicht zum vorveröffentlichten Stand der Technik gehörenden Patentanmeldung der Anmelderin (vgl. DE-OS 3 334 455.).

Man erhält die Verbindungen der Formel (IV) wenn man Cyanamid-Derivate der Formel (VI)

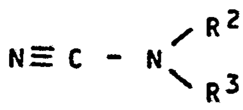

(VI)

in welcher

$R^2$ und $R^3$ die oben angegebenen Bedeutungen haben,

mit Hydroxylamin-Derivaten der Formel (VII)

$$H_2N\text{-}OR^1 \qquad (VII)$$

in welcher

$R^1$ die oben angegebene Bedeutung hat,

bzw. mit Hydrochloriden von Hydroxylamin-Derivaten der Formel (VII)

gegebenenfalls in Gegenwart von Verdünnungsmitteln, wie z. B. Ethanol, Propanol oder Butanol, bei Temperaturen zwischen 20 °C und 120 °C umsetzt und gegebenenfalls die Umsetzungsprodukte mit Säureakzeptoren, wie z. B. Ammoniak, Kaliumcarbonat oder Natriumhydroxid, behandelt.

Die Cyanamid-Derivate der Formel (VI ) sind zum Teil bekannt (vergl. J. Chem. Soc. 1953, 1725). Man erhält die Verbindungen der Formel ( VI) im wesentlichen nach folgenden Synthesewegen:

(a) durch Umsetzung von Alkalimetall- oder Erdalkalimetall-Salzen von Cyanamid - wie z. B. Natriumcyanamid oder Calciumcyanamid - mit Chlor-hetarenen der Formel (VIII)

$$Cl\text{-}R^3 \qquad (VIII)$$

in welcher

$R^3$ die oben angegebene Bedeutung hat,

und gegebenenfalls anschließend - wenn $R^2$ nicht für Wasserstoff steht - mit Halogenverbindungen der Formel (IX)

$$T\text{-}R^2 \qquad (IX)$$

in welcher

$R^2$ für einen gegebenenfalls substituierten Rest aus der Reihe Alkyl, Alkenyl, Alkinyl und Aralkyl steht, und

T für Chlor, Brom oder Jod steht,

gegebenenfalls in Gegenwart von inerten Verdünnungsmitteln, wie z. B. Aceton, Acetonitril oder Dimethylformamid, bei Temperaturen zwischen 0 °C und 100 °C.

Nach Einengen und Auflösen des Rückstandes in Wasser können die Cyanamid-Derivate der Formel (VI) durch Ansäuern, z. B. mit Salzsäure, ausgefällt und durch Absaugen isoliert werden.

Alternativ erhält man die Verbindungen der Formel (VI)

(b) für den Fall, daß $R^3$ für einen substituierten Pyrimidinylrest steht, durch Umsetzung von Cyanoguanidin ('Dicyandiamid') mit ß-Dicarbonylverbindungen, wie Acetylaceton (vergl. J. Chem. Soc. 1953, 1725 - 1730), Acetessigsäureestern (vergl. J. Prakt. Chem. 77 (1908), 542 und J. Chem.Soc. 1948, 586) oder Malonsäureestern (vergl. DE-PS 158 591).

Die aus Acetessigsäureestern bzw. Malonsäureestern erhaltenen 2-Cyanamino-4-hydroxy-6-methyl- bzw. -4,6-dihydroxy-pyrimidine können auf bekannte Weise durch Umsetzung mit Alkylierungsmitteln, wie z. B. Dimethyl- oder Diethylsulfat, gegebenenfalls in Gegenwart von Verdünnungsmitteln, wie z. B. Wasser, Methanol, Ethanol, n- und iso-Propanol, Aceton, Dioxan oder Dimethylformamid, und in Gegenwart von Säurebindemitteln, wie z. B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z. B. Acetanhydrid oder Acetylchlorid acyliert und nach der Alkylierung mit wässrigen Säuren oder Basen wieder entacyliert.

In einem weiteren Alternativverfahren erhält man die Verbindungen der Formel (VI ), wenn man

(c) Amino-hetarene der Formel (X)

$$H_2N-R^3 \qquad (X)$$

in welcher

$R^3$ die oben angegebene Bedeutung hat,

mit Carbonylisothiocyanaten der Formel (XI)

$$R^7-\overset{O}{\overset{\|}{C}}-N=C=S \qquad (XI)$$

in welcher

$R^7$ für Ethoxy oder Phenyl steht,

gegebenenfalls in Gegenwart eines inerten Verdünnungsmittels, wie z. B. Aceton, Acetonitril oder Toluol, bei Temperaturen zwischen 0 °C und 100 °C umsetzt, die hierbei gebildeten Carbonylthioharnstoffe der Formel (XII)

$$R^7-\overset{O}{\overset{\|}{C}}-NH-\overset{S}{\overset{\|}{C}}-NH-R^3 \qquad (XII)$$

in welcher

$R^3$ und $R^7$ die oben angegebenen Bedeutung haben,

gegebenenfalls nach Einengen durch Absaugen isoliert und mit wässrigen Alkalimetall- oder Erdalkalimetallhydroxidlösungen, wie z. B. Natronlauge, gegebenenfalls in Gegenwart eines organischen Lösungsmittels, wie z. B. Tetrahydrofuran oder Dioxan, bei Temperaturen zwischen 0 °C und 120 °C umsetzt und die nach Ansäuern, z. B. mit Salzsäure, kristallin erhaltenen Thioharnstoffe der Formel (XIII)

$$H_2N-\overset{S}{\overset{\|}{C}}-NH-R^3 \qquad (XIII)$$

in welcher

$R^3$ die oben angegebene Bedeutung hat,

durch Absaugen isoliert und mit Metallverbindungen, welche Schwefelwasserstoff binden können, wie z. B. mit Blei(II)-acetat, Kupfer(II)-acetat, Quecksilber-(II)-acetat oder Eisen(II)-acetat, in Gegenwart von wässrigen Alkalimetall- oder Erdalkalimetall-hydroxidlösungen, wie z. B. Natronlauge, bei Temperaturen zwischen 20 °C und 100 °C umsetzt, nach Ende der Umsetzung filtriert und das Filtrat mit einer Säure, wie z. B. Essigsäure, ansäuert. Die hierbei kristallin anfallenden Produkte der Formel (VI) können durch Absaugen isoliert werden.

Die Ausgangsstoffe für die vorausgehend unter (a), (b) und (c) beschriebenen Herstellungsverfahren für die Cyanamid-Derivate der Formel (VI) sind bekannt und/oder können nach an sich bekannten Verfahren hergestellt werden.

Hierzu gehören die Chlor-hetarene der Formel (VIII) (vergl. J. Chem. Soc. (c) 1966, 2031; Chem. Pharm. Bull. 11

(1963), 1382-1388 und Arch.Pharm. 295, (1962), 649-657), die Halogenverbindungen der Formel (IX) (handelsübliche Chemikalien), die Amino-hetarene der Formel (X) (vgl. Chem. Pharm.Bull.11, (1963),1382-1388; J.Chem.Soc. 1946, 81 und US-PS 4 299 960) und die Carbonylisothiocyanate der Formel (XI) (vgl. J.Heterocycl.Chem. 5, (1968), 837 und US-PS 4 160 037).

Die beim erfindungsgemäßen Verfahren(a) weiter als Ausgangsstoffe zu verwendenden Verbindungen sind durch die Formel (III) allgemein definiert. In Formel (III) haben Q und R vorzugsweise bzw. insbesondere die gleichen Bedeutungen, wie sie oben im Rahmen der Substituentendefinition der Formel (I) vorzugsweise bzw. insbesondere bevorzugt genannt sind und

M steht vorzugsweise wie auch insbesondere für Wasserstoff, Natrium oder Kalium.

Als Beispiele für die Ausgangsstoffe der Formel (III) seien genannt:
Methanol, Ethanol, Propanol, Isopropanol, Butanol, Isobutanol, Allylalkohol, Propargylalkohol, Benzylalkohol, Glycolsäuremethylester, Milchsäuremethylester, Phenol, 2-, 3- und 4-Fluor-phenol, 2-, 3- und 4-Chlorphenol, 2-, 3-und 4-Brom-phenol, 4-Iod-phenol, 2,3-Dichlorphenol, 2,4-Dichlor-phenol, 2,5-Dichlor-phenol, 4-Cyanophenol, 2-, 3- und 4-Nitro-phenol, 4-Chlor-3-nitro-phenol, 3-Chlor-4-nitro-phenol, Brenzkatechin (1,2-Dihydroxy-benzol), Resorcin (1,3-Dihydroxy-benzol), Hydrochinon (1,4-Dihydroxy-benzol), 2-, 3- und 4-Hydroxy-benzoesäure, 2-, 3- und 4-Methyl-phenol, 3,4-Dimethyl-phenol, 3,5-Dimethylphenol, 4-Isopropyl-phenol, 4-tert.-Butyl-phenol, 4-Chlor-3,5-dimethyl-phenol, 4-Hydroxy-benzylalkohol, 3-Methyl-4-nitro-phenol, 4-Chlor-3-methyl-phenol, 3- und 4-Trifluormethylphenol, 4-Hydroxy-phenylessigsäure-methylester, 4-(1-Methyl-1-phenyl-ethyl)-phenol, 4-Cyclohexyl-phenol, 3- und 4-Hydroxy-benzoesäure-methylester und -ethylester, 2-, 3- und 4-Methoxy-phenol, 4-Trifluormethoxy-phenol, 4-Methylthio-phenol, 3-Methyl-4-methylthio-phenol, 4-Trifluormethylthio-phenol, 3-Dimethylamino-phenol, 3-Methyl-4-dimethylamino-phenol, 4-Methyl-3-dimethylamino-phenol, 4-Acetylamino-phenol, 3-Hydroxy- und 4-Hydroxy-benzaldehyd, 4-Hydroxy-acetophenon, 4-Hydroxy-benzophenon, 4-Hydroxy-biphenyl, 4,4'-Bis-hydroxy-biphenyl, 1-Naphthol, 2-Naphthol, 3-Hydroxy-biphenyl, 4-Hydroxy-azobenzol, 4-Hydroxy-diphenylamin, 3-Hydroxy-diphenylamin, 3-Phenoxy-phenol, 4-Phenoxy-phenol, 4-(2,4-Dichlor-phenoxy-phenol, 4-(4-Trifluormethyl-phenoxy)-phenol, 4-(3,5-Dichlor-2-pyridoxy)-phenol, 4-(3-Chlor-5-trifluormethyl-2-pyridoxy)-phenol, Methanthiol,Ethanthiol,Propanthiol, 1-Methyl-ethanthiol, Butanthiol, 1-Methyl-propanthiol, 2-Methyl-propanthiol, Phenylmethanthiol, Thiophenol, 4-Chlor-thiophenol,4-Methylthiophenol,2-Amino-thiophenol,3-Amino-thiophenol, 4-Aminophenol, 2-Amino-thiophenol, 3-Amino-thiophenol, 4-Methoxy-thiophenol, 2-Mercapto-benzoesäure und 4-Hydroxythiophenol, sowie die Natrium und Kalium-Salze dieser Verbindungen.

Die Ausgangsstoffe der Formel (III) sind bekannte, weitgehend handelsübliche Produkte.

Das beim erfindungsgemäßen Verfahren (b) als Ausgangsstoff zu verwendende Benzol-1,2-disulfonsäuredichlorid der Formel (V) ist bereits bekannt (vgl. J.Org.Chem. 31, (1966), 3289-3292).

Die beim erfindungsgemäßen Verfahren (b) als Ausgangsstoffe zu verwendenden Oxyguanidin-Derivate sind durch die Formel (IV) allgemein definiert. In Formel (IV) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.
Beispiele für Verbindungen der Formel (IV) wurden bereits weiter oben im Zusammenhang mit der Beschreibung der Ausgangsstoffe für Verfahren (a) genannt. Die Herstellung der Ausgangsstoffe der Formel (IV) wurde bereits im Zusammenhang mit der Beschreibung der Ausgangsstoffe für Verfahren (a) beschrieben.

Die beim erfindungsgemäßen Verfahren (b) weiter als Ausgangsstoffe zu verwendenden Verbindungen sind durch die Formel (III) allgemein definiert. In Formel (III) haben Q und R vorzugsweise bzw. insbesondere die gleichen Bedeutungen, wie sie oben im Rahmen der Substituentendefinition der Formel (I) vorzugsweise bzw. als insbesondere bevorzugt genannt sind und M steht vorzugsweise wie auch insbesondere für Wasserstoff, Natrium oder Kalium.

Beispiele für Verbindungen der Formel (III) wurden bereits weiter oben im Zusammenhang mit der Beschreibung der Ausgangsstoffe für Verfahren (a) genannt.

Das erfindungsgemäße Verfahren (a) zur Herstellung der neuen Verbindungen der Formel (I) wird vorzugsweise unter Verwendung von Verdünnungsmitteln durchgeführt. Als solche

kommen praktisch alle organischen Solventien in Betracht. Hierzu gehören gegebenenfalls halogenierte Kohlenwasserstoffe, wie z.B. Methylenchlorid, Chloroform, Toluol und Chlorbenzol, Ether, wie z.B. Diethylether, Tetrahydrofuran, Dioxan und 1,2-Dimethoxyethan, Nitrile, wie z.B. Acetonitril und Propionitril sowie Dimethylformamid, Dimethylacetamid, Dimethylsulfoxid und Sulfolan.
Die unter Formel (III) fallenden Alkohole, wie z.B. Methanol, Ethanol, Propanol und Isopropanol können gegebenenfalls - soweit sie als Reaktionskomponenten eingesetzt werden - auch als Verdünnungsmittel verwendet werden.

Verfahren (a) wird gegebenenfalls in Gegenwart von Basen durchgeführt. Als solche kommen praktisch alle üblicherweise verwendeten Säurebindemittel in Betracht. Hierzu gehören insbesondere Metall-hydroxide, -carbonate, -hydride und -alkoholate, wie z.B. Natrium-, Kalium- und Calcium-hydroxid, Kaliumcarbonat, Natrium- und Calciumhydrid, Natrium-methylat, -ethylat und -propylat, Kaliummethylat, -ethylat und tert.-Butylat sowie Aluminiumisopropylat , ferner Stickstoffheterocyclen, wie z.B. Pyrazol.
Die bei Verfahren (a) gebildeten Produkte werden gegebenenfalls mit Säuren behandelt. Es kommen verschiedenste Protonendonatoren in Betracht, wie z.B. Salzsäure, Schwefelsäure, Phosphorsäure, Ameisensäure, Essigsäure, Kohlensäure, Natriumhydrogencarbonat, Ammoniumcarbonat und Wasser.
Die Reaktionstemperaturen können beim erfindungsgemäßen Verfahren (a) innerhalb eines größeren Bereiches variiert werden. Im allgemeinen arbeitet man bei Temperaturen zwischen 0°C und 150°C, vorzugsweise zwischen 10°C und 100°C. Das erfindungsgemäße Verfahren wird im allgemeinen bei Normaldruck durchgeführt.

Zur Durchführung von Verfahren (a) setzt man je Mol Benzodisultame der Formel (II) im allgemeinen zwischen 1 und 100 Mol, vorzugsweise zwischen 1 und 10 Mol der Verbindung der Formel (III) ein. Die Reaktionskomponenten werden gewöhnlich bei Raumtemperatur oder unter Außenkühlung zusammengegeben und das Reaktionsgemisch wird, gegebenenfalls bei erhöhter Temperatur, bis zum Reaktionsende gerührt. Die Aufarbeitung und Isolierung der neuen Verbindungen erfolgt nach üblichen Methoden:

Beispielsweise wird das Reaktionsgemisch - gegebenenfalls nach Verrühren mit einem mit Wasser praktisch nicht mischbaren Lösungsmittel, wie z.B. Methylenchlorid - mit verdünnter Salzsäure und mit Wasser gewaschen, getrocknet, filtriert und eingeengt. Das im Rückstand verbliebene Produkt der Formel (I) wird durch Verreiben mit einem geeigneten organischen Lösungsmittel, wie z.B. Ethanol zur Kristallisation gebracht und durch Absaugen isoliert.

Das erfindungsgemäße Verfahren (b) zur Herstellung der neuen Verbindungen der Formel (I) wird vorzugsweise unter Verwendung von Verdünnungsmitteln durchgeführt. Als solche kommen praktisch alle inerten organischen, vorzugsweise aprotisch polare Solventien in Betracht. Hierzu gehören gegebenenfalls substituierte Kohlenwasserstoffe, wie z.B. Methylenchlorid, Chloroform, 1,2-Dichlorethan,

Toluol, Xylol und Chlorbenzol, Nitrile, wie z.B. Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (b) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylaminopyridin.

Die Reaktionstemperaturen können bei Verfahren (b) innerhalb eines größeren Bereiches variiert werden. Im allgemeinen arbeitet man zwischen -80 und +150°C, vorzugsweise zwischen -30 und +100°C. Das erfindungsgemäße Verfahren (b) wird im allgemeinen bei Normaldruck durchgeführt.

Zur Durchführung von Verfahren (b) setzt man je Mol Oxyguanidin-Derivat der Formel (IV) im allgemeinen zwischen 1 und 2 Mol, vorzugsweise zwischen 1,0 und 1,2 Mol Benzol-1,2-disulfonsäure-dichlorid der Formel (V ) und anschließend zwischen 1 und 100 Mol, vorzugsweise zwischen 5 und 10 Mol der Verbindung der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung kann auf übliche Weise durchgeführt werden; beispielsweise indem man gegebenenfalls einengt und/oder mit einem mit Wasser praktisch nicht mischbaren organischen Lösungsmittel, wie z.B. Methylenchlorid, verdünnt, mit verdünnter Salzsäure und mit Wasser wäscht, trocknet, filtriert und einengt. Das im Rückstand verbliebene Produkt der Formel (I) wird durch Verreiben mit einem geeigneten organischen Lösungsmittel, wie z.B. Ethanol, zur Kristallisiation gebracht und durch Absaugen isoliert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne

Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Oelpalm-, Kakao-, Beerenfrucht-und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele:

Beispiel 1

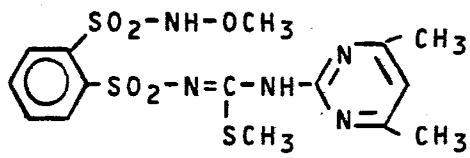

(Verfahren (b))

6,9g (0,025 Mol) Benzol-1,2-disulfonsäure-dichlorid werden portionsweise bei -20°C zu einer Mischung von 4,9g (0,025 Mol) N'-(4,6-Dimethyl-pyrimidin-2-yl)-N''-methoxy-guanidin, 6g (0,087 Mol) Pyridin und 80 ml Methylenchloird gegeben. Man rührt 3 Stunden bei -20°C und 15 Stunden bei +20°C nach.

Dann wird das Reaktionsgemisch mit 1,2 g (0,025 Mol) Methylmercaptan versetzt und man rührt 5 Stunden bei 20°C nach. Anschließend wird das Lösungsmittel abgezogen und der Rückstand mit wenig Alkohol verrieben. Das hierbei kristallin anfallende Produkt wird durch Absaugen isoliert. Man erhält 8 g (71 % der Theorie) N'-(4,6-Dimethyl-pyrimidin-2-yl)-N''-(2-methoxyaminosulfonylphenylsulfonyl)-S-methyl-iso-thioharnstoff vom Schmelzpunkt 201°C.

Beispiel 2

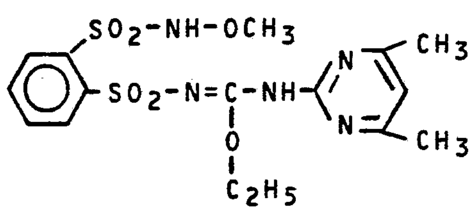

(Verfahren (a))

Eine Lösung von 7g (0,0175 Mol) der Verbindung der Strukturformel

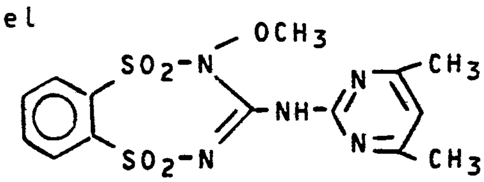

in 30 ml Ethanol wird 4 Stunden unter Rückfluß erhitzt. Dann wird teilweise eingeengt und das kristallin anfallende Produkt durch Absaugen isoliert.

Nach dem Umkristallisieren aus Acetonitril erhält man 5,4g (71 % der Theorie) N'-(4,6-Dimethyl-pyrimidin-2-yl)-N''-(2-methoxyaminosulfonylphenylsulfonyl)-O-ethyl-iso-harnstoff vom Schmelzpunkt 170-171°C.

Beispiel 3

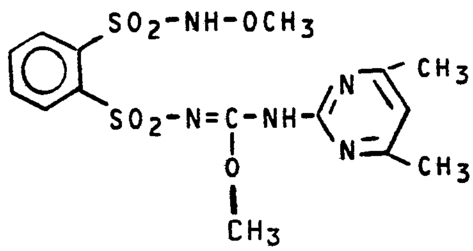

(Verfahren (a))

Eine Mischung aus 4,8g (0,012 Mol) der Verbindung nachstehender Strukturformel,

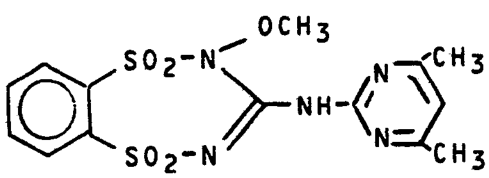

0,82g (0,012 Mol) Pyrazol und 30 ml Methanol wird 18 Stunden bei 20°C gerührt. Das kristallin anfallende Produkt wird durch Absaugen isoliert und getrocknet.

Man erhält 5,1g (97 % der Theorie) N'-(4,6-Dimethyl-pyrimidin-2-yl)-N''-(2-methoxyaminosulfonylphenylsulfonyl)-O-methyl-iso-harnstoff vom Schmelzpunkt 163 - 165°C.

Nach den in den vorausgehenden Beispielen exemplarisch beschriebenen Verfahren können die in der nachstehenden Tabelle 2 aufgeführten Verbindungen der Formel (I) hergestellt werden:

Tabelle 2:

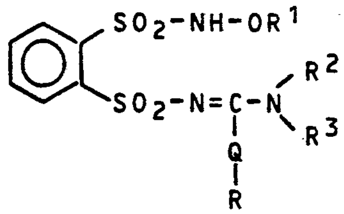

(I)

| Bsp.Nr. | R | $R^1$ | $R^2$ | $R^3$ | Q | Schmelzpunkt (°C) |
|---|---|---|---|---|---|---|
| 4 | 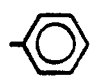 | $-CH_3$ | H | 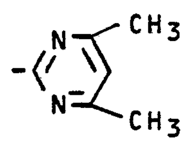 | S | 188 (Zers.) |
| 5 | 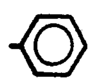 | $-CH_3$ | H | 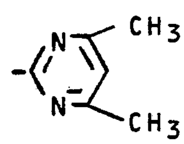 | O | |
| 6 | 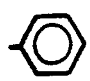 | $-C_4H_9$-n | H | 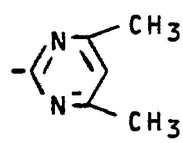 | S | 141 |
| 7 | $-C_2H_5$ | $-C_3H_7$-i | H | 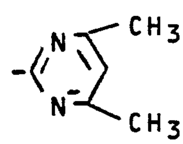 | O | 161 |
| 8 | $-CH_3$ | $-C_3H_7$-n | H | 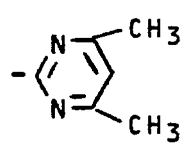 | O | |
| 9 | $-CH_3$ | $-C_8H_{17}$ | H | 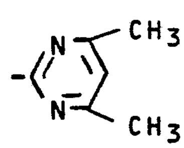 | S | |

Tabelle 2 (Fortsetzung)

| Bsp. Nr. | R | $R^1$ | $R^2$ | $R^3$ | Q | Schmelzpunkt (°C) |
|---|---|---|---|---|---|---|
| 10 | 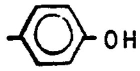 | 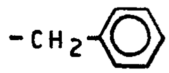 | H | 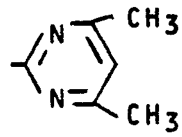 | S | |
| 11 | $-CH_3$ | $-C_2H_5$ | H | 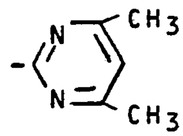 | O | |
| 12 | $-CH_3$ | $-C_2H_5$ | H | 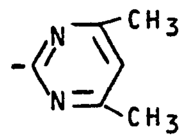 | S | |
| 13 | 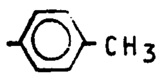 | $-C_2H_5$ | H | 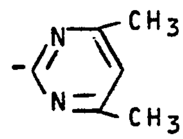 | S | |
| 14 | 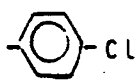 | $-C_2H_5$ | H | 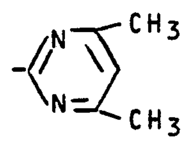 | O | |
| 15 | $-CH_3$ | $-CH_2COOC_2H_5$ | H | 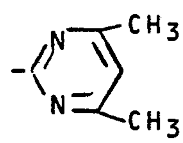 | S | |
| 16 | $-C_2H_5$ | $-CH(CH_3)_2$ | H | 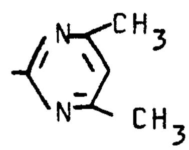 | O | 161 |
| 17 | $-C_2H_5$ | $-C_3H_7$-n | H | 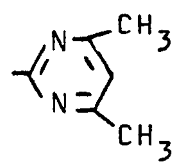 | O | 131 |
| 18 | $-C_2H_5$ | $-C_2H_5$ | H | 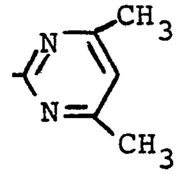 | O | 209 |

<u>Tabelle 2</u> (Fortsetzung)

| Bsp. Nr. | R | $R^1$ | $R^2$ | $R^3$ | Q | Schmelzpunkt (°C) |
|---|---|---|---|---|---|---|
| 19 | $-CH_3$ | $-CH_3$ | H | 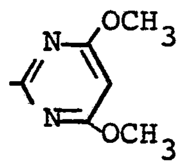 | O | |
| 20 | $-C_2H_5$ | $-CH_3$ | H | 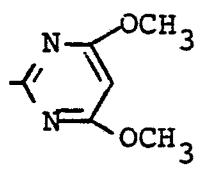 | O | |
| 21 | 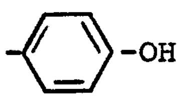 | $-C_2H_5$ | H | 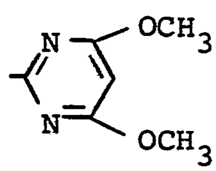 | S | |
| 22 | $-C_2H_5$ | $-C_2H_5$ | H | 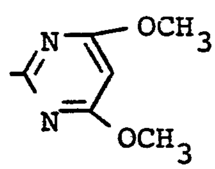 | O | |
| 23 | $-CH_3$ | $-CH_3$ | H | 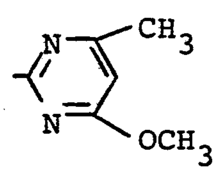 | O | |
| 24 | $-C_2H_5$ | $-CH_3$ | H | 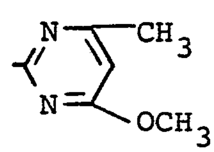 | O | |

Tabelle 2 (Fortsetzung)

| Bsp. Nr. | R | $R^1$ | $R^2$ | $R^3$ | Q | Schmelzpunkt (°C) |
|---|---|---|---|---|---|---|
| 25 | 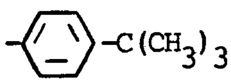 | $-CH_3$ | H | 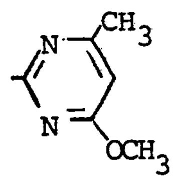 | O | |
| 26 | 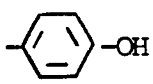 | $-CH_3$ | H | 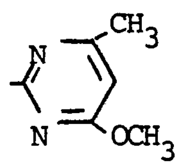 | S | |
| 27 | $-CH_3$ | $-C_2H_5$ | H | 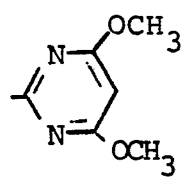 | O | |
| 28 | $-CH_3$ | $-C_2H_5$ | H | 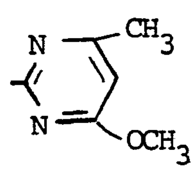 | O | |
| 29 | $-CH_3$ | $-CH_2CH{=}CH_2$ | H | 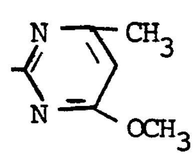 | O | |
| 30 | $-CH_3$ | $-CH_3$ | H | 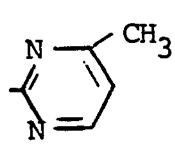 | O | |
| 31 | $-C_2H_5$ | $-CH_3$ | H | 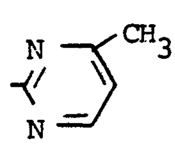 | O | |

Tabelle 2 (Fortsetzung)

| Bsp. Nr. | R | $R^1$ | $R^2$ | $R^3$ | Q | Schmelzpunkt (°C) |
|---|---|---|---|---|---|---|
| 32 | 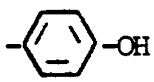 | $-CH_3$ | H | 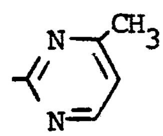 | S | |
| 33 | $-CH_3$ | $-C_2H_5$ | H | 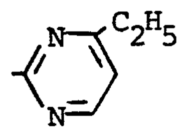 | O | |
| 34 | $-CH_3$ | $-CH_3$ | H | 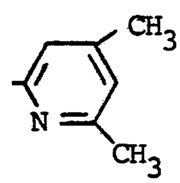 | O | |
| 35 | $-CH_3$ | $-CH_3$ | H | 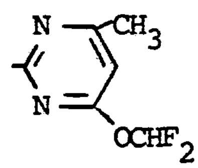 | O | |
| 36 | $-CH_3$ | $-CH_3$ | H | 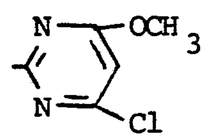 | O | |
| 37 | $-CH_3$ | $-CH_3$ | H | 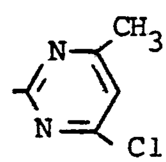 | O | |
| 38 | $-CH_3$ | $-CH_3$ | $-CH_3$ | 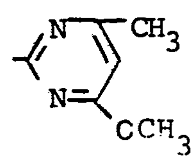 | O | |
| 39 | $-CH_3$ | $-CH_3$ | H | 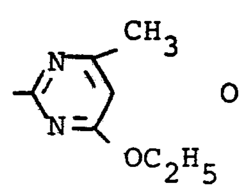 | O | |

Herstellung von Ausgangsstoffen der Formel (II)

Beispiel (II-1)

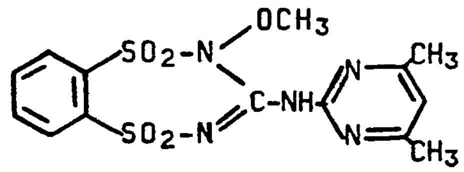

14 g (0,05 Mol) Benzol-1,2-disulfonsäure-dichlorid werden portionsweise bei -20 °C zu einer Mischung von 10 g (0,05 Mol) N'-(4,6-Dimethylpyrimidin-2-yl)-N''-methoxyguanidin, 12 g (0,15 Mol) Pyridin und 100 ml Methylenchlorid gegeben. Man rührt 3 Stunden bei -20 °C und 15 Stunden bei +20 °C nach.

Dann wird das Reaktionsgemisch mit eisgekühlter verdünnter Salzsäure und Eiswasser gewaschen. Die Methylenchloridlösung wird getrocknet und eingeengt. Der Rückstand wird mit Ethanol verrieben. Der hierbei kristallin angefallene Rückstand wird durch Absaugen isoliert.

Man erhält 11,5 g (58 % der Theorie) der Verbindung der oben angegebenen Strukturformel vom Schmelzpunkt 158 °C (Zers.).

Die Aufarbeitung des Reaktionsgemisches kann auch in der Weise erfolgen, daß man nach beendeter Reaktion vollständig eindampft, den Rückstand mit Ethanol verreibt und das Disultam obiger Struktur durch Absaugen isoliert.

Nach dem in vorausgehenden Beispiel exemplarisch beschriebenen Verfahren können die in der nachstehenden Tabelle 3 aufgeführten Verbindungen der Formel (II)hergestellt werden:

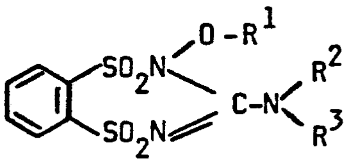

(II)

Tabelle 3

| Beispiel Nr. | $R^1$ | $R^2$ | $R^3$ | Schmelzpunkt(°C) |
|---|---|---|---|---|
| (II-2) | $-C_2H_5$ | H | 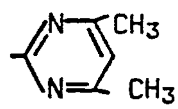 | 104 (Zers.) |
| (II-3) | $-C_3H_7i$ | H | 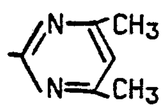 | amorph |
| (II-4) | $-C_3H_7n$ | H | 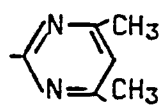 | 134 |
| (II-5) | $-C_4H_9n$ | H | 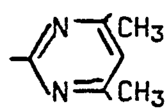 | 179(Zers.) |
| (II-6) | $-C_8H_{17}n$ | H | 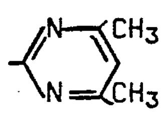 | 164 |
| (II-7) | $-CH_2$- 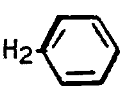 | H | 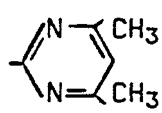 | 198 |
| (II-8) | $-CH_2CH_2$- 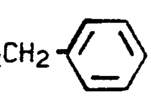 | H | 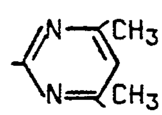 | |

Tabelle 3 (Fortsetzung)

| Bsp. Nr. | $R^1$ | $R^2$ | $R^3$ | Schmelzpunkt (°C) |
|---|---|---|---|---|
| (II-9) | $-CH_2CH(CH_3)_2$ | H | 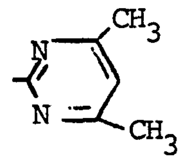 | |
| (II-10) | $-CH_2CH{=}CH_2$ | H | 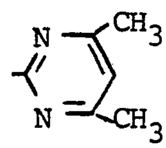 | 180 |
| (II-11) | $-CH_3$ | $-CH_3$ | 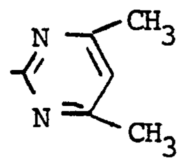 | |
| (II-12) | $-CH_3$ | $-CH_3$ | 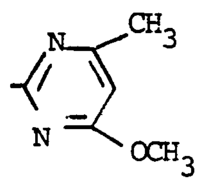 | |
| (II-13) | $-CH_3$ | H | 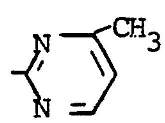 | |
| (II-14) | $-CH_3$ | H | 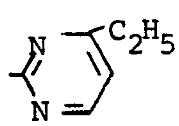 | |
| (II-15) | $-CH_2COOC_2H_5$ | H | 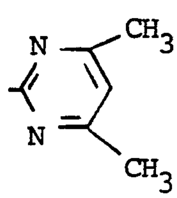 | 210 (Zers.) |

Tabelle 3 (Fortsetzung)

| Bsp. Nr. | $R^1$ | $R^2$ | $R^3$ | Schmelzpunkt (°C) |
|---|---|---|---|---|
| (II-16) | $-CH(CH_3)_2$ | H | 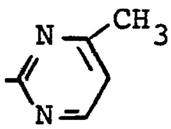 | |
| (II-17) | $-CH_3$ | H | 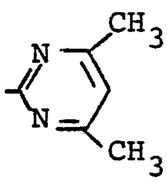 | |
| (II-18) | $-CH_3$ | H | 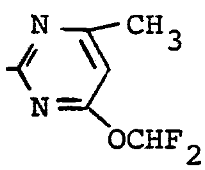 | |
| (II-19) | $-CH_3$ | H | 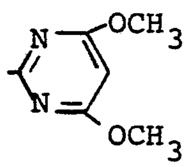 | |
| (II-20) | $-C_2H_5$ | H | 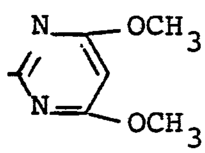 | |
| (II-21) | $-CH_2CH(CH_3)_2$ | H | 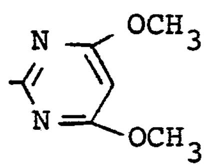 | |
| (II-22) | $-CH_3$ | H | 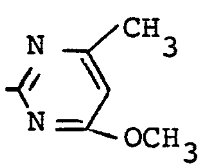 | 151 |

Tabelle 3 (Fortsetzung)

| Bsp. Nr. | $R^1$ | $R^2$ | $R^3$ | Schmelzpunkt (°C) |
|---|---|---|---|---|
| (II-23) | $-CH_2CH=CH_2$ | H | 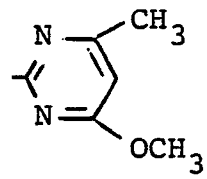 | |
| (II-24) | $-CH_3$ | H | 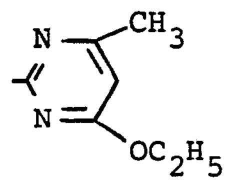 | |

Herstellung von Ausgangsstoffen der Formel (IV)

Beispiel (IV-1)

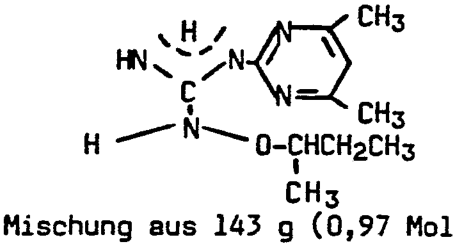

Eine Mischung aus 143 g (0,97 Mol) 2-Cyanamino-4,6-dimethyl-pyrimidin, 94,3 g (1,06 Mol) O-sec.-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57% der Theorie) N'-(4,6-Dimethyl-pyrimidin-2-yl)-N''-sec.-butoxy-guanidin vom Schmelzpunkt 52°C.

Analog können die in der nachstehenden Tabelle 4 aufgeführten Verbindungen der Formel (IV) hergestellt werden:

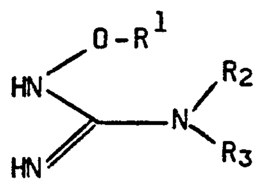

(IV)

Tabelle 4

| Beispiel Nr. | $R^1$ | $R^2$ | $R^3$ | Schmelzpunkt(°C) |
|---|---|---|---|---|
| (IV-2) | $-CH_2CH(CH_3)_2$ | H | 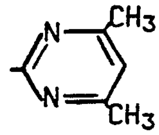 | 78 |
| (IV-3) | $-CH_2CH=CH_2$ | H | 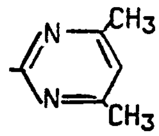 | 103 |
| (IV-4) | $-CH(CH_3)_2$ | H | 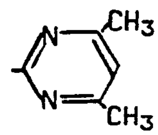 | 84 |
| (IV-5) | $-CH_2-CH_2-$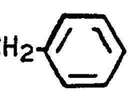 | H | 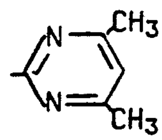 | $n_D^{24}=1,5776$ |
| (IV-6) | $-C_4H_9-n$ | H | 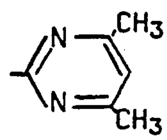 | (Oel) |
| (IV-7) | $-C_8H_{17}-n$ | H | 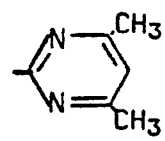 | 58 |
| (IV-8) | 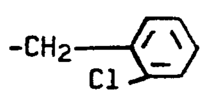 | H | 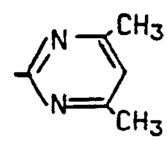 | 102-103 |
| (IV-9) | $-CH_2CH_2CH_2Cl$ | H | 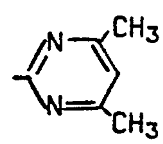 | 137 |
| (IV-10) | 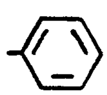 | H | 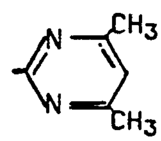 | 189-192 (Zers.) |

Tabelle 4-Fortsetzung

| Beispiel Nr. | $R^1$ | $R^2$ | $R^3$ | Schmelzpunkt(°C) |
|---|---|---|---|---|
| (IV-11) | $-CH_2COOCH_3$ | H | 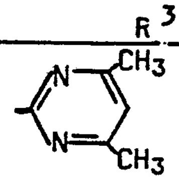 | 148-149 |
| (IV-12) | $-CH_2COOC_2H_5$ | H | 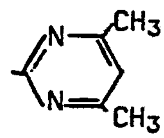 | 98- 99 |
| (IV-13) | $-CH(CH_3)-COOCH_3$ | H | 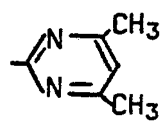 | 147-148 |
| (IV-14) | 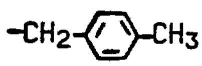 | H | 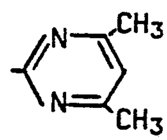 | 85- 86 |
| (IV-15) | 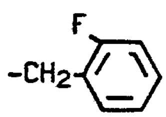 | H | 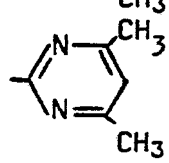 | 114 - 116 |
| (IV-16) | 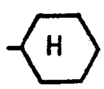 | H | 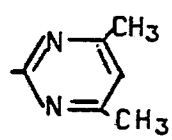 | |
| (IV-17) | 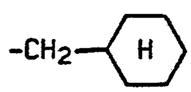 | H | 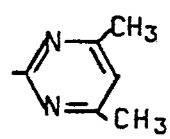 | |
| (IV-18) | $-CH_2CON(CH_3)_2$ | H | 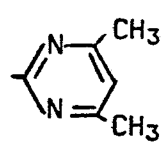 | |
| (IV-19) | $-CH_2OCH_3$ | H | 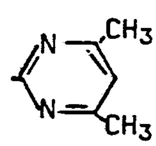 | |

Tabelle 4- Fortsetzung

| Beispiel Nr. | $R^1$ | $R^2$ | $R^3$ | Schmelzpunkt (°C) |
|---|---|---|---|---|
| (IV-20) | $-CH_2SCH_3$ | H | 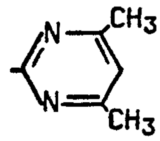 | |
| (IV-21) | 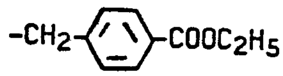 | H | 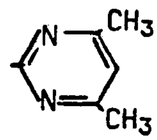 | 138 |
| (IV-22) | $-CH_2CF_3$ | H | 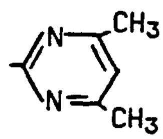 | |
| (IV-23) | 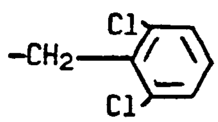 | H | 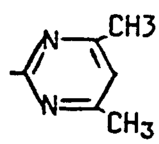 | 140-145 |
| (IV-24) | 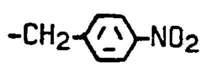 | H | 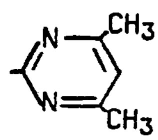 | 170 - 172 |
| (IV-25) | $-CH_3$ | H | 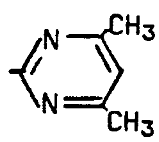 | 134-136 |
| (IV-26) | $-C_2H_5$ | H | 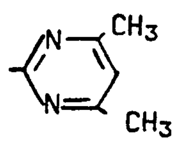 | 88 |
| (IV-27) | 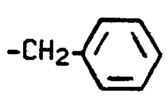 | H | 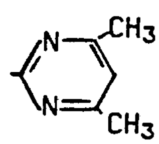 | 102 |
| (IV-28) | $-CH_3$ | $-CH_3$ | 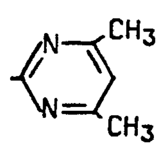 | 95 |

Tabelle 4 - Fortsetzung

| Beispiel Nr. | $R^1$ | $R^2$ | $R^3$ | Schmelzpunkt(°C) |
|---|---|---|---|---|
| (IV-29) | $-CH_3$ | $CH_3$ | 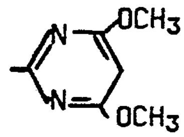 | 135 |
| (IV-30) | $-CH_3$ | H | 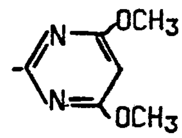 | 122 |
| (IV-31) | $-CH_3$ | H | 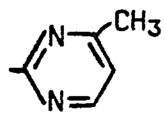 | 152 |
| (IV-32) | $-CH_3$ | H | 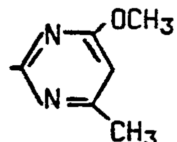 | 126 |
| (IV-33) | $-CH_3$ | H | 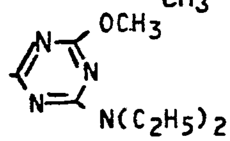 | 112 |
| (IV-34) | $-CH_3$ | H | 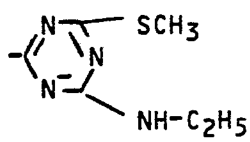 | 117 |
| (IV-35) | $-CH_2CH_2CH_3$ | H | 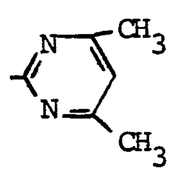 | 54 |
| (IV-36) | $-CH_2COOCH(CH_3)_2$ | H | 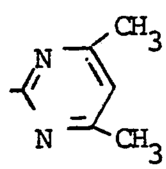 | 112 |
| (IV-37) | $-C_2H_5$ | H | 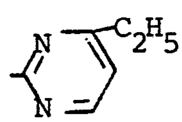 | |

Tabelle 4 (Fortsetzung)

| Beispiel Nr. | $R^1$ | $R^2$ | $R^3$ | Schmelzpunkt (°C) |
|---|---|---|---|---|
| (IV-38) | 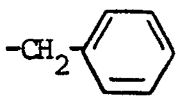 | H | 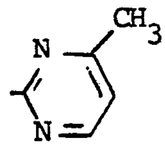 | 150 |
| (IV-39) | 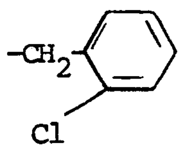 | H | 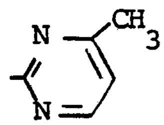 | 140 |
| (IV-40) | $-CH_3$ | H | 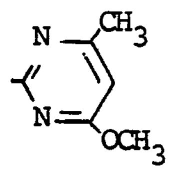 | 126 |
| (IV-41) | $-CH_3$ | $-CH_3$ | 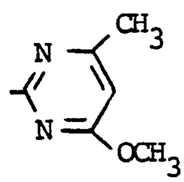 | 135 |
| (IV-42) | $-CH_3$ | H | 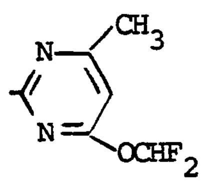 | |
| (IV-43) | $-C_2H_5$ | H | 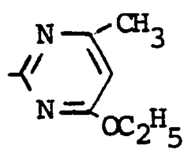 | |

Tabelle 4 (Fortsetzung)

| Beispiel Nr. | $R^1$ | $R^2$ | $R^3$ | Schmelzpunkt (°C) |
|---|---|---|---|---|
| (IV-44) | $-CH_3$ | H | 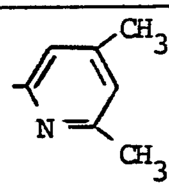 | 110 |
| (IV-45) | $-CH_3$ | H | 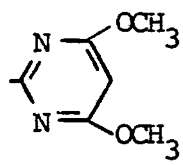 | 122 |
| (IV-46) | $-\underset{CH_3}{CH}CH_2CH_3$ | H | 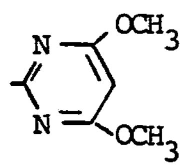 | 68 |
| (IV-47) | $-CH_2CH(CH_3)_2$ | H | 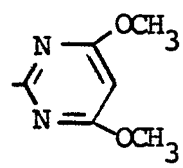 | 76 |
| (IV-48) | $-CH_3$ | H | 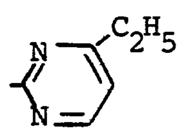 | 98 |
| (IV-49) | $-C_2H_5$ | H | 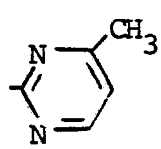 | 95 |
| (IV-50) | $-CH_2-$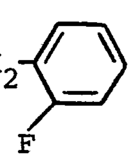 | H | 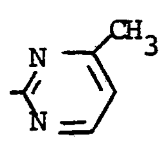 | 205 |

Tabelle 4 (Fortsetzung)

| Beispiel Nr. | $R^1$ | $R^2$ | $R^3$ | Schmelzpunkt (°C) |
|---|---|---|---|---|
| (IV-51) | $-CH_2CH_2CH_2Cl$ | H | 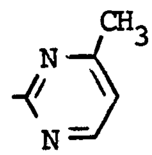 | 102 |
| (IV-52) | $-CH_2COOC_2H_5$ | H | 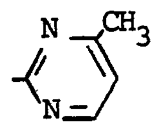 | |
| (IV-53) | $-CH_2CH=CH_2$ | H | 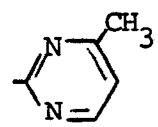 | |
| (IV-54) | $-C_4H_9-n$ | H | 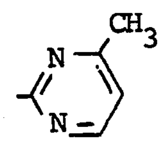 | |
| (IV-55) | $-\underset{CH_3}{CH}CH_2CH_3$ | H | 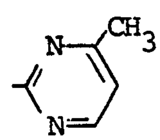 | |
| (IV-56) | $-CH_3$ | H | 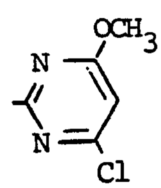 | 112 |
| (IV-57) | $-CH_3$ | H | 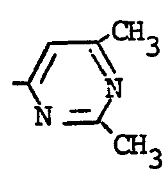 | 143 |

Tabelle 4 (Fortsetzung)

| Beispiel Nr. | $R^1$ | $R^2$ | $R^3$ | Schmelzpunkt (°C) |
|---|---|---|---|---|
| (IV-58) | 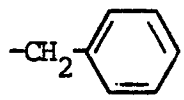 | H | 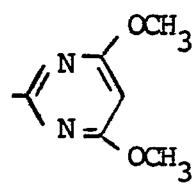 | 74 |
| (IV-59) | 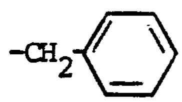 | H | 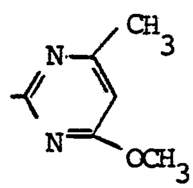 | $n_{20}^{D}$: 1,5645 |
| (IV-60) | 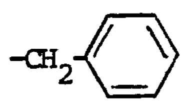 | H | 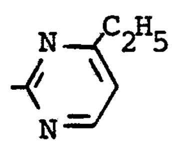 | 112 |

Beispiel (VI-1)

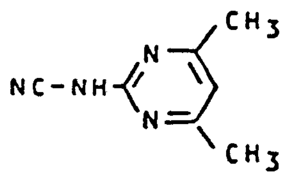

Ein Gemisch aus 42 g (0,5 Mol) Cyanoguanidin ('Dicyandiamid') und 50 g (0,5 Mol) 2,4-Pentandion ('Acetylaceton') wird 15 Stunden auf 120°C erhitzt. Dann wird nach Abkühlen das Reaktionsgemisch mit 500 ml Wasser versetzt und die Lösung bei 0°C bis 10°C mit Salzsäure angesäuert. Das hierbei kristallin angefallene Produkt wird durch Absaugen isoliert. Man erhält 51,8 g (70 % der Theorie) 2-Cyanamino-4,6-dimethyl-pyrimidin vom Schmelzpunkt 205°C.

Beispiel (VI-2)

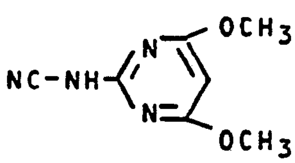

Eine auf 100°C erhitzte Lösung von 24 g (0,427 Mol) Kaliumhydroxid in 100 ml Wasser wird bei 100°C unter Rühren zu einer Mischung von 9,2g (0,043 Mol) 4,6-Dimethoxypyrimidin-2-yl-thioharnstoff und 70 ml Wasser gegeben. Man rührt 2 Minuten bei 100°C nach und gibt dann eine auf 100°C erwärmte Lösung von 16,2g (0,05 Mol) Blei-II-acetat in 30 ml Wasser hinzu. Man erhitzt noch 5 Minuten unter Rückfluß, kühlt dann auf 0°C bis 5°C ab und versetzt die wässrige Lösung mit 30ml Eisessig. Das hierbei kristallin ausfallende Produkt wird durch Absaugen isoliert.
Man erhält 6,3g (81,5 % der Theorie) 2-Cyanamino-4,6-dimethoxy-pyrimidin vom Schmelzpunkt 202°C.

Nach dem in den vorausgehenden Beispielen (VI-1) und (VI-2) exemplarisch beschriebenen Verfahren können auch die in der nachstehenden Tabelle 5 aufgeführten Verbindungen der Formel (VI) hergestellt werden:

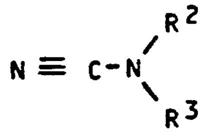 (VI)

Tabelle 5

| Bsp. Nr. | $R^2$ | $R^3$ | Schmelzpunkt(°C) |
|---|---|---|---|
| (VI-3) | H | 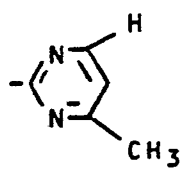 | 203(Zer.) |
| (VI-4) | H | 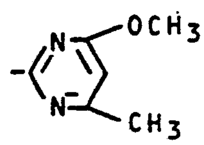 | 258 |
| (VI-5) | H | 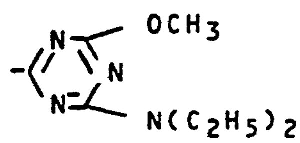 | 114 |
| (VI-6) | H | 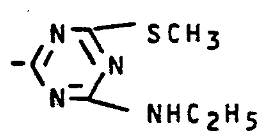 | |
| (VI-7) | H | 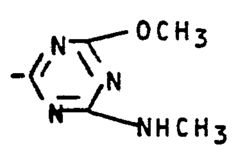 | 210 |
| (VI-8) | H | 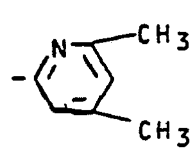 | 221 |

Tabelle 5 (Fortsetzung)

| Bsp. Nr. | $R^2$ | $R^3$ | Schmelzpunkt (°C) |
|---|---|---|---|
| (VI-9) | H | 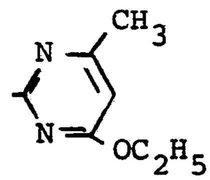 | |
| (VI-10) | H | 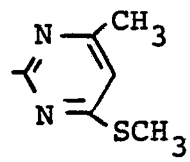 | |
| (VI-11) | H | 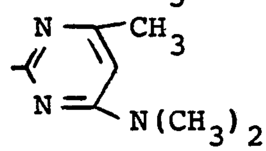 | |
| (VI-12) | H | 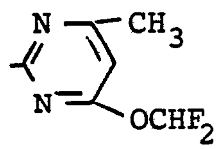 | 174 |
| (VI-13) | H | 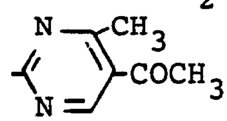 | 174 |
| (VI-14) | H | 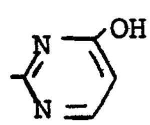 | 300 (Zers.) |
| (VI-15) | H | 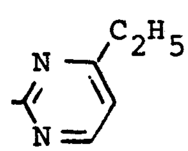 | 146 |

Tabelle 5 (Fortsetzung)

| Bsp. Nr. | $R^2$ | $R^3$ | Schmelzpunkt (°C) |
|---|---|---|---|
| (VI-16) | H | 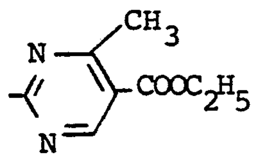 | 126 |
| (VI-17) | H | 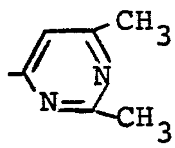 | 234 |
| (VI-18) | H | 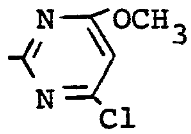 | 200 |
| (VI-19) | H | 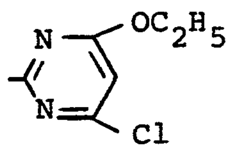 | |
| (VI-20) | H | 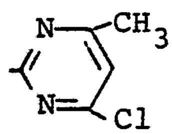 | |
| (VI-21) | H | 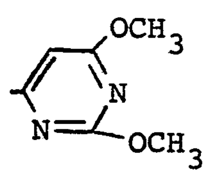 | |
| (VI-22) | H | 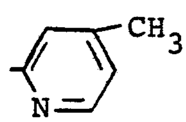 | 250 |

Tabelle 5 (Fortsetzung)

| Bsp. Nr. | $R^2$ | $R^3$ | Schmelzpunkt (°C) |
|---|---|---|---|
| (VI-23) | H | 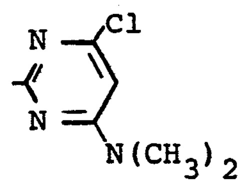 | |
| (VI-24) | H | 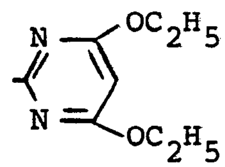 | |

2-(Alkyl-cyano-amino)-pyrimidine der Formel (VI) können beispielsweise wie folgt hergestellt werden:

Beispiel (VI-25)

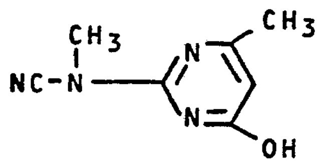

12,6g (0,1 Mol) Dimethylsulfat werden zu einer Lösung von 15 g (0,1 Mol) 2-Cyanamino-4-hydroxy-6-methyl-pyrimidin und 4,1g (0,1 Mol) Natriumhydroxid in 60 ml Wasser tropfenweise gegeben, wobei die Reaktionstemperatur von 20°C auf 40°C steigt. Nach zweistündigem Rühren bei 20°C wird das kristallin angefallene Produkt durch Absaugen isoliert.
Man erhält 11,1g (68 % der Theorie) 2-(Methyl-cyan-amino)-4-hydroxy-6-methyl-pyrimidin vom Schmelzpunkt 290°C.

Analog erhält man:
Beispiel (VI-26)

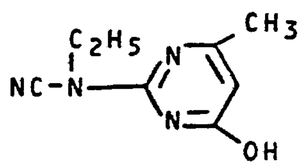

Fp: 215°C bis 220°C.

Beispiel (VI-27)

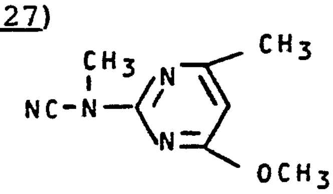

127,5 g (1 Mol) Dimethylsulfat werden zu einer Lösung von 75 g (0,5 Mol) 2-Cyanamino-4-hydroxy-6-methyl-pyrimidin und 44 g (1,1 Mol) Natriumhydroxid in 750 ml Wasser tropfenweise gegeben,

wobei die Reaktionstemperatur von 20°C auf 35°C ansteigt. Nach zwölfstündigem Rühren bei 20°C wird durch Zugabe von Natronlauge ein pH-Wert zwischen 9 und 10 eingestellt und das kristallin angefallene Produkt durch Absaugen isoliert.

Man erhält 13 g (15 % der Theorie) 2-(Methyl-cyano-amino)-4-methoxy-6-methyl-pyrimidin vom Schmelzpunkt 123°C.

Analog erhält man

Beispiel (VI-28)

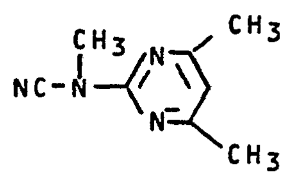

Fp: 104°C.

Beispiel (VI-29)

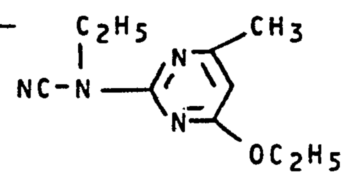

Fp: 71°C.

Herstellung der Ausgangsstoffe der Formel (XII)

Beispiel (XII-1)

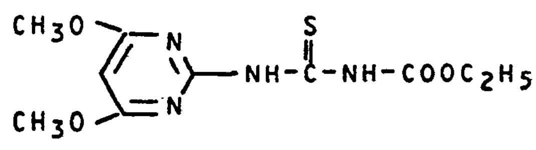

Eine Mischung aus 15,5g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60°C gerührt. Dann wird auf 10°C abgekühlt, und das kristallin angefallene Produkt durch Absaugen isoliert.

Man erhält 22,5 g (79% der Theorie) 1-(Ethoxycarbonyl)-3-(4,6-dimethoxy-pyrimidin-2-yl)-thioharnstoff vom Schmelzpunkt 194°C (Zers.).

Nach dem im vorausgehenden Beispiel exemplarisch beschriebenen Verfahren können die in der nachstehenden Tabelle 6 aufgeführten Verbindungen der Formel (XII) hergestellt werden:

$$R^7-\overset{O}{\overset{\|}{C}}-NH-\overset{S}{\overset{\|}{C}}-NH-R^3 \qquad (XII)$$

Tabelle 6

| Beispiel Nr. | $R^7$ | $R^3$ | Schmelzpunkt (°C) |
|---|---|---|---|
| (XII-2) | 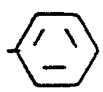 | 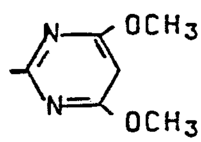 | 189 |
| (XII-3) | 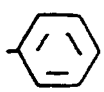 | 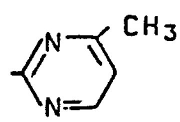 | 198-9 (Zers.) |
| (XII-4) | $-OC_2H_5$ | 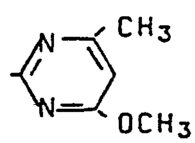 | 217 |
| (XII-5) | 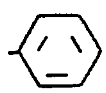 | 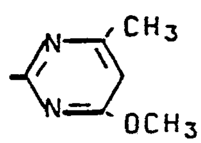 | 190 |
| (XII-6) | $-OC_2H_5$ | 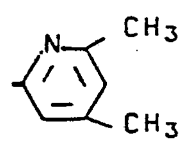 | 140 |
| (XII-7) | 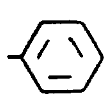 | 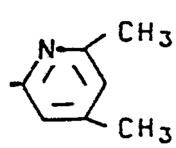 | 145 |

Tabelle 6 (Fortsetzung)

| Bsp. Nr. | $R^7$ | $R^3$ | Schmelzpunkt (°C) |
|---|---|---|---|
| (XII-8) | $-OC_2H_5$ | 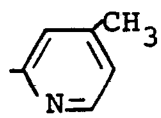 | 119 |
| (XII-9) | 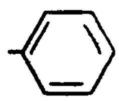 | 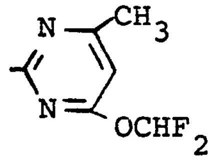 | 182 |
| (XII-10) | $-OC_2H_5$ | 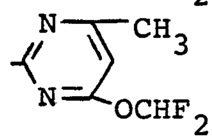 | 185 |
| (XII-11) | $-OC_2H_5$ | 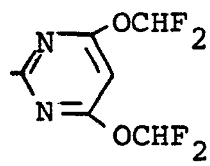 | 173 |
| (XII-12) | $-OC_2H_5$ | 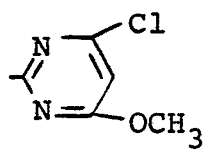 | 162 |
| (XII-13) | $-OC_2H_5$ | 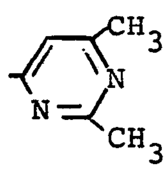 | 169 |
| (XII-14) | 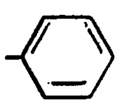 | 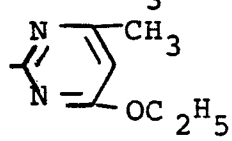 | 156 |
| (XII-15) | $-OC_2H_5$ | 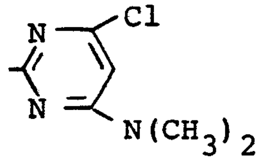 | 168 |

Tabelle 6 (Fortsetzung)

| Bsp. Nr. | $R^7$ | $R^3$ | Schmelzpunkt (°C) |
|---|---|---|---|
| (XII-16) | 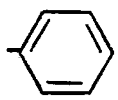 | 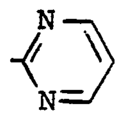 | 173 |
| (XII-17) | 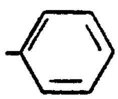 | 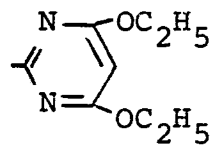 | 179 |
| (XII-18) | $-OC_2H_5$ | 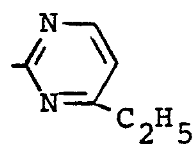 | |
| (XII-19) | 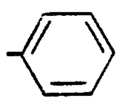 | 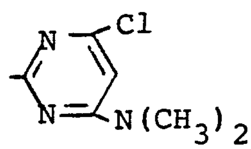 | 168 |
| (XII-20) | $-OC_2H_5$ | 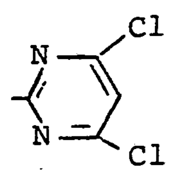 | 132-136 |
| (XII-21) | $-OC_2H_5$ | 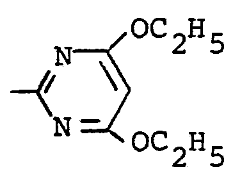 | |
| (XII-22) | 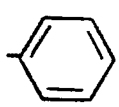 | 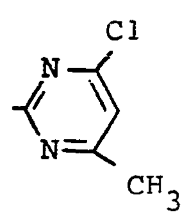 | 144 |

Herstellung der Ausgangsstoffe der Formel (XIII)

Beispiel (XIII-1)

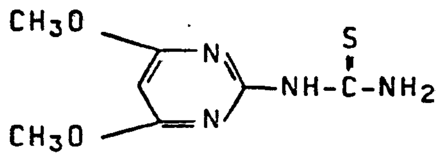

Eine Mischung von 5,0 g (0,0175 Mol) 1-(Ethoxycarbonyl)-3-(4,6-dimethoxy-pyrimidin-2-yl)-thioharnstoff, von 4,0 g (0,1 Mol) Natriumhydroxid und von 100 ml Wasser wird 2 Tage bei 20°C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94% der Theorie) 4,6-Dimethoxypyrimidin-2-yl-thioharnstoff vom Schmelzpunkt 245-8°C (Zers.).

Nach dem im vorausgehenden Beispiel exemplarisch beschriebenen Verfahren können die in der nachstehenden Tabelle 7 aufgeführten Verbindungen der Formel (XIII) hergestellt werden:

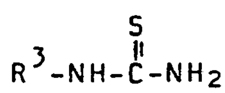

(XIII)

Tabelle 7

| Beispiel Nr. | $R^3$ | Schmelzpunkt (°C) |
| --- | --- | --- |
| (XIII-2) | 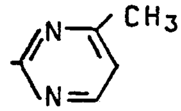 | 264-265 (Zers.) |
| (XIII-3) | 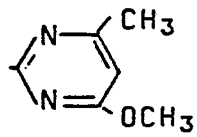 | 205-207 (Zers.) |
| (XIII-4) | 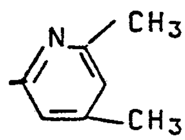 | 259-260 (Zers.) |
| (XIII-5) | 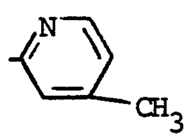 | 214-215 |
| (XIII-6) | 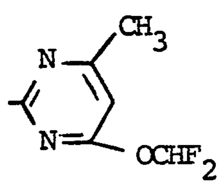 | 192-194 |
| (XIII-7) | 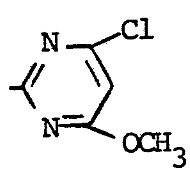 | 225-227 (Zers.) |
| (XIII-8) | 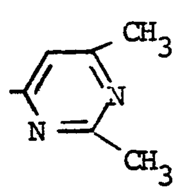 | 248 |

Tabelle 7 (Fortsetzung)

| Beispiel Nr. | $R^3$ | Schmelzpunkt (°C) |
|---|---|---|
| (XIII-9) | 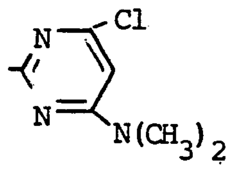 | |
| (XIII-10) | 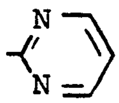 | 263 |
| (XIII-11) | 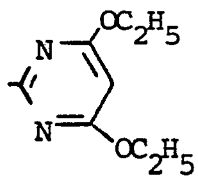 | |
| (XIII-12) | 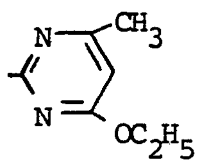 | |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die Verbindungen der Herstellungsbeispiele (1) und (4) eine deutlich bessere Wirksamkeit gegen mono- und dikotyle Unkräuter als die Verbindung (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigen z.B. die folgenden Verbindungen der Herstellungsbeispiele (1) und (4) eine deutlich bessere Wirksamkeit gegen mono- und dikotyle Unkräuter als die Verbindung (A).

Patentansprüche

1.) 1-(2-Oxyaminosulfonylphenylsulfonyl)-3-heteroaryl-iso-(thio)-harnstoffe der allgemeinen Formel (I)

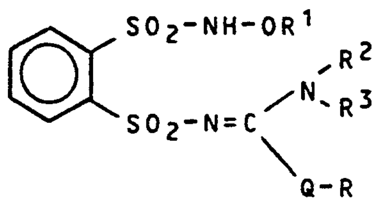

(I)

in welcher

R für einen gegebenenfalls substituierten Rest aus der Reihe Alkyl, Alkenyl, Alkinyl, Cycloalkyl-alkyl, Cycloalkyl, Aralkyl, Aryl und Hetaryl steht,

$R^1$ für einen gegebenenfalls substituierten Rest aus der Reihe Alkyl, Alkenyl, Alkinyl, Cycloalkyl, Cycloalkyl-alkyl, Aralkyl und Aryl steht,

$R^2$ für Wasserstoff oder für einen gegebenenfalls substituierten Rest aus der Reihe Alkyl, Alkenyl, Alkinyl und Aralkyl steht,

$R^3$ für einen gegebenenfalls substituierten und/oder gegebenenfalls anellierten sechsgliedrigen aromatischen Heterocyclus welcher wenigstens ein Stickstoffatom enthält, steht und

Q für Sauerstoff oder Schwefel steht,

sowie Addukte der Verbindungen der Formel (I) mit starken Säuren.

2.) Verfahren zur Herstellung von 1-(2-Oxyaminosulfonyl-phenylsulfonyl)-3-heteroaryl-iso(thio)-harnstoffen der allgemeinen Formel (I) gemäß Anspruch 1, dadurch gekennzeichnet, daß man

(a) Benzodisultame der Formel (II)

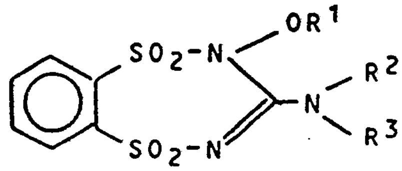

(II)

in welcher

$R^1$, $R^2$ und $R^3$ die oben angegebenen Bedeutungen haben,

mit Verbindungen der Formel (III)

$$M - Q - R \qquad (III)$$

in welcher

Q und R die oben angegebenen Bedeutungen haben und

M für Wasserstoff oder ein Metalläquivalent steht,

gegebenenfalls in Gegenwart von Basen und gegebenenfalls in Gegenwart von Verdünnungsmitteln umsetzt und gegebenenfalls die hierbei erhaltenen Produkte mit Säuren behandelt,

oder daß man

(b) Oxyguanidin-Derivate der Formel (IV)

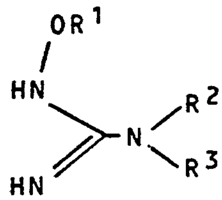

(IV)

in welcher $R^1$, $R^2$ und $R^3$ die oben angegebenen Bedeutungen haben,

zunächst mit Benzol-1,2-disulfonsäuredichlorid der Formel (V)

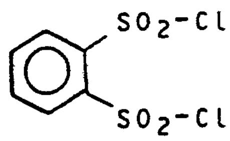

(V)

in Gegenwart von Säureakzeptoren und gegebenenfalls in Gegenwart von Verdünnungsmitteln umsetzt und anschließend, ohne Isolierung des Reaktionsproduktes der Formel (II)

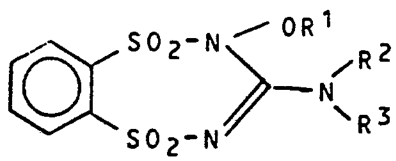

(II)

in welcher

$R^1$, $R^2$ und $R^3$ die oben angegebenen Bedeutungen haben,

die Reaktionsmischung mit Verbindungen der Formel (III)

$$M - Q - R \qquad (III)$$

in welcher

Q und R die oben angegebenen Bedeutungen haben und
M für Wasserstoff oder ein Metalläquivalent steht,

gegebenenfalls in Gegenwart von Basen und in Gegenwart von Verdünnungsmitteln umsetzt und gegebenenfalls die hierbei erhaltenen Produkte mit Säuren behandelt.

3.) Herbizide Mittel, gekennzeichnet durch einen Gehalt an mindestens einem 1-(2-Oxyaminosulfonylphenylsulfonyl)-3-heteroaryl-iso(thio)-harnstoff der allgemeinen Formel (I) gemäß Anspruch 1.

4.) Verwendung von 1-(2-Oxyaminosulfonylphenylsulfonyl)-3-heteroaryl-iso(thio)-harnstoffen der allgemeinen Formel (I) gemäß Anspruch 1 zur Bekämpfung von unerwünschtem Pflanzenwachstum.

5.) Verfahren zur Herstellung von herbiziden Mitteln, dadurch gekennzeichent, daß man 1-(2-Oxyaminosulfonylphenylsulfonyl)-3-heteroaryl-iso(thio)-harnstoffe der allgemeinen Formel (I) gemäß Anspruch 1 mit Streckmitteln und/oder oberflächenaktiven Mitteln vermischt.